(19) Europäisches Patentamt
European Patent Office
Office européen des brevets

(11) EP 1 084 711 B1

(12) EUROPEAN PATENT SPECIFICATION

(45) Date of publication and mention of the grant of the patent:
**11.05.2005 Bulletin 2005/19**

(21) Application number: **99922538.6**

(22) Date of filing: **28.05.1999**

(51) Int Cl.[7]: **A61K 45/00**, A61K 31/38 // C07D333:68

(86) International application number:
**PCT/JP1999/002820**

(87) International publication number:
**WO 1999/062555 (09.12.1999 Gazette 1999/49)**

# (54) REMEDIES FOR THE TREATMENT OF ITCHING CONTAINING PGD2 ANTAGONISTS

MITTEL ZUR LINDERUNG VON JUCKREIZ, ENTHALTEND PGD2- ANTAGONISTEN

REMEDES CONTRE LE PRURIT RENFERMANT DES ANTAGONISTES DE PGD2

(84) Designated Contracting States:
**AT BE CH CY DE DK ES FI FR GB GR IE IT LI LU MC NL PT SE**

(30) Priority: **03.06.1998 JP 15433298**

(43) Date of publication of application:
**21.03.2001 Bulletin 2001/12**

(73) Proprietor: **SHIONOGI & CO., LTD.**
**Osaka-shi, Osaka 541-0045 (JP)**

(72) Inventor: **ARIMURA, Akinori**
**Osaka-shi, Osaka 558-0041 (JP)**

(74) Representative: **Hayes, Adrian Chetwynd et al**
**Boult Wade Tennant,**
**Verulam Gardens**
**70 Gray's Inn Road**
**London WC1X 8BT (GB)**

(56) References cited:
**EP-A- 0 409 392**
**EP-A- 0 837 052**
**WO-A-98/25919**
**WO-A1-97/00853**
**JP-A- 3 204 815**

- **TSURI T ET AL: "BICYCLOU2.2.1HEPTANE AND 6,6-DIMETHYLBICYCLOU3.1.1HEPTANE DERIVATIVES: ORALLY ACTIVE, POTENT, AND SELECTIVE PROSTAGLANDIN D2 RECEPTOR ANTAGONISTS" JOURNAL OF MEDICINAL CHEMISTRY, AMERICAN CHEMICAL SOCIETY. WASHINGTON, US, vol. 40, 1997, pages 3504-3507, XP002058538 ISSN: 0022-2623**
- **NAKAJIMA M ET AL: "EFFECTS OF PROSTAGLANDIN D2 AND ITS ANALOGUE, BW245C, ON INTRAOCULAR PRESSURE IN HUMANS" GRAEFE'S ARCHIVE FOR CLINICAL AND EXPERIMENTAL OPHTHALMOLOGY, SPRINGER VERLAG, XX, vol. 229, no. 4, 1991, pages 411-413, XP000650937 ISSN: 0721-832X**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 084 711 B1

## Description

Technical Field

The present invention relates to use of a compound in the manufacture of a medicament for the prevention or treatment of itching, said compound being a prostaglandin $D_2$ ($PGD_2$) receptor antagonist.

Background Art

$PGD_2$ is a major prostanoid released from mast cells in which it is produced through $PGG_2$ and $PGH_2$ from arachidonic acid by the action of cyclooxygenase activated by immunological or unimmunological stimulation. $PGD_2$ has been known to cause allergic disorders such as allergic rhinitis and allergic conjunctivitis because it shows various physiological effects such as induction of nasal obstruction, vasodilator effect, wandering of eosinophils.

Accordingly, $PGD_2$ receptor antagonists have been thought to be useful for the treatment thereof (WO97/00853).

Moreover, a large quantity of $PGD_2$ is also released from macrophages, so $PGD_2$ may play a role in causing an inflammatory response independent of allergy.

On the other hand, itching has been known to be accompanied by diseases such as atopic dermatitis, urticaria, allergic rhinitis, allergic conjunctivitis, as well as inflammatory responses such as swelling.

Moreover, the action accompanied by itching, for example scratching, knocking, may worsen the condition of the above-mentioned diseases. Therefore, the development of a compound for the treatment of itching has been desired, which is further expected to be a pharmaceutical composition for the prevention or treatment of diseases secondarily caused by the action against itching, for example cataracts, retinal detachment, inflammation, infection, dysgryphia.

At present, antihistaminic agents are used as therapeutic agents for itching. They show an effect on swelling, but the effect against itching is by no means sufficient. Thus, it is suggested in Allergology Int., 1997, 46, 117 - - 124 that itching may be caused by a mediator other than histamine.

A $PGD_2$ receptor antagonist used in the present invention has been known to be useful for the treatment of allergic conditions caused by $PGD_2$ such as rhinitis and the like (WO97/00853). No positive data concerning the prevention or treatment of itching has been described.

On the other hand, it is described in J. Pharmacol. Exp. Ther., 279, 137-142, 1996 that instillation of $PGD_2$ in guinea pigs induces itching, which is inhibited by a $PGD_2$ receptor antagonist, BWA868C. However it is not described that a $PGD_2$ receptor antagonist is useful for the treatment of itching caused by allergy. Further described is that a $PGD_2$ receptor antagonist, BWA868C, cannot inhibit itching caused by antigens at all, which is similar to disease models.

On the other hand, it is described that Ramatroban, inhibiting the contraction of smooth muscle of bronchus caused by $TXA_2$ or $PGD_2$ stimulation, is efficacious against contact dermatitis or atopic dermatitis mediated by delayed allergy (WO97/44031). However, Ramatroban described in said specification is a $TXA_2$ receptor antagonist, but not a $PGD_2$ receptor antagonist. Moreover, the therapeutic effect of Ramatroban against atopic dermatitis is based on the suppression of swelling caused by the delayed-type allergy reaction. Thus, the suppression effect against itching is not described. Therefore, it is not suggested that a $PGD_2$ receptor antagonist of the present invention suppresses itching and is useful for the treatment of atopic dermatitis.

## Disclosure of Invention

$PGD_2$, a mediator mass-produced through allergy reaction, is supposed to play an important role as a mediator in itching. Indeed, we have discovered through an experiment using mice that a $PGD_2$ receptor antagonist is efficacious against itching to accomplish the present invention. Therefore, the present invention provides use of a compound of the formula (IA-a):

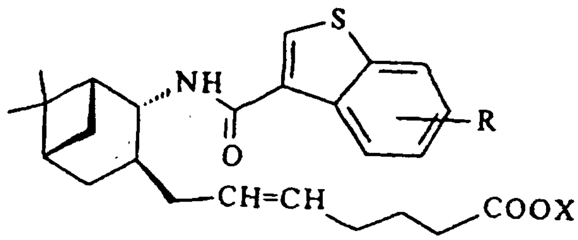

**(IA-a)**

wherein R is hydrogen, fluoro or hydroxyl, X is hydrogen and the double bond on the α chain has Z configuration, a pharmaceutical acceptable salt thereof or a hydrate thereof, in the manufacture of a medicament for the treatment or prevention of itching.

The $PGD_2$ receptor antagonist used in the present invention has an activity of preventing or treating itching and so it can be used for a pharmaceutical composition for the prevention or treatment of itching. The term "itching" used in the present specification means itching caused by an allergic reaction or a non-allergic reaction.

Allergic reaction means reactions caused by the activation of mast cells, basophil and the like due to the reaction of an antigen with the antigen-specific IgE and the delayed-type allergy reaction such as contact dermatitis. Non-allergic reaction means a reaction which is independent of IgE and caused by mast cells, basophils and the like activated, e.g. by a chemical substance.

The $PGD_2$ receptor antagonist used in the present invention suppresses itching derived from allergic reaction or non-allergic reaction and is useful for the prevention or treatment of the accompanying inflammation such as atopic dermatitis, urticaria, allergic conjunctivitis, allergic rhinitis or contact dermatitis.

Moreover, the $PGD_2$ receptor antagonist is useful for the prevention or treatment of a secondary disease such as cataracts, retinal separation, inflammation, infection, dysgryphia, which is caused by an action accompanied by itching, for example, scratching, knocking.

## Best Mode for Carrying Out the Invention

The $PGD_2$ receptor antagonist used in the present invention has an activity of preventing or treating itching. Preferably, the compound for use in the present invention is of the formula (IA-a), wherein R is hydroxy and X is hydrogen.

Through the present specification, the group of the formula in the compound of the formula (IA-a) :

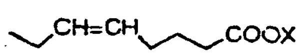

wherein X is as defined above,
is referred to as α chain and the group of the formula:

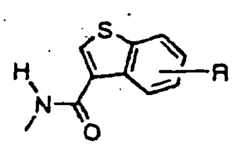

wherein R is as defined above,
is referred to as ω chain.

As mentioned above, the double bond on the α-chain of the compound used in the present invention has Z configuration. In the compounds of formula (IA-a) used in the present invention R is hydrogen, fluoro or hydroxyl and X is hydrogen. Preferably, R is hydroxy and X is hydrogen.

Even more preferably, the compound used in the present invention is of the formula (IA-a-1), wherein X is H.

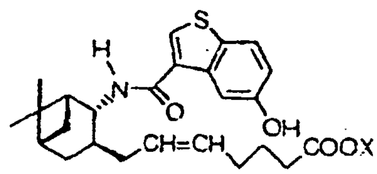

IA-a-I

A preferred $PGD_2$ receptor antagonist has a high $PGD_2$ antagonistic activity and a high selectivity. Another preferred has a low agonistic activity. For example, a preferred antagonist has a $PGD_2$ binding inhibitory activity ($IC_{50}$ value) of 1000 nM or less, 100 nM or less or especially 10 nM or less. The $PGD_3$ binding inhibitory activity ($IC_{50}$ value) can be calculated in accordance with the experiment 1 of the present specification.

Each term used in the present specification is defined below.

The term "alkyl" means C1-C6 straight or branched alkyl, for example, methyl, ethyl, n-propyl, i-propyl, n-butyl, i-butyl, s-butyl, t-butyl, n-pentyl, i-pentyl, neopentyl, t-pentyl, t-pentyl, hexyl.

The term "alkoxy" means C1-C6 straight or branched alkoxy, for example, methoxy, ethoxy, n-propoxy, i-propoxy, n-butoxy.

The term "halogen" is fluoro, chloro, bromo or iodo.

The term "acyl" of the term "acyloxy" means C1-C9 acyl derived from aliphatic carboxylic acid, for example, formyl, acetyl, propionyl, butyryl, valeryl.

The term "acyloxy" means acyloxy derived from the above "acyl", for example, acetoxy, propionyloxy, butyryloxy, valeryloxy.

The term "aryl" means C6-C14 aromatic monocyclic group or aromatic condensed ring, for example, phenyl, naphthyl (e.g., 1-naphthyl or 2-naphthyl). anthryl (e.g., 1-anthryl, 2-anthryl or 9-anthryl).

The term "arylsulfonyloxy" means arylsulfonyloxy derived from "aryl", for example, arylsulfonyloxy, 1-nathylsulfonyloxy, 1-anthrylsulfonyloxy.

The substituent of "aryl" includes alkyl, alkoxy, halogen, hydroxy

Examples of salts of the compound of the formula (IA-a) includes those formed with an alkali metal (e.g., lithium, sodium or potassium), an alkali earth metal (e.g., calcium), an organic base (e.g., tromethamine, trimethylamine, triethylamine, 2-aminobutane, t-butylamine, diisopropylethylamine, n-butylmethylamine, cyclohexylamine, dicyclohexylamine, N-isopropylcyclohexylamine, furfurylamine, benzylamine, methylbenzylamine, dibenzylamine, N,N-dimethylbenzylamine, 2-chlorobenzylamine, 4-methoxybenzylamine, 1-naphthalenemethylamine, diphenylbenzylamine, triphenylamine, 1-naphthylamine, 1-aminoanthracene, 2-aminoanthracene, dehydroabiethylamine, N-methylmorpholine or pyridine), an amino acid (e.g., lysine or arginine). These salts can be formed in accordance with the usual methods.

The hydrates of the compound of the formula (IA-a) may be coordinated with water molecules in an optional proportion.

General processes for the preparation of the compound of the formula (IA-a) are illustrated as follows. Any substituent interfering with a reaction may be protected in advance with a protecting group and deprotected in a suitable step.

Process 1

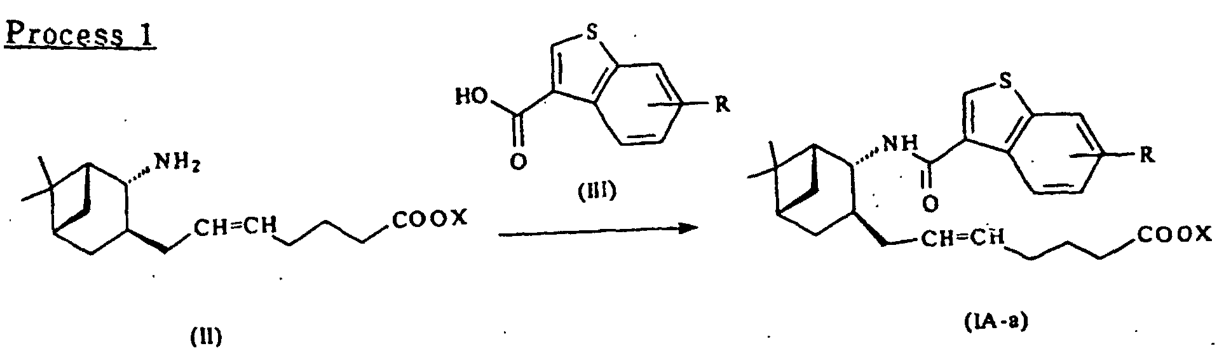

wherein X and R are as defined above and the double bond on the α chain has Z configuration.

The compound of the formula (IA-a) as shown in the above process 1 can be prepared by reacting the carboxylic acid of the formula (III) or the reactive derivative with an amino compound of the formula (II)

A starting compound (II) is described in the Japanese Patent Publication (Kokoku) No. 23170/1994.

The carboxylic acid of the formula (III) includes 5-fluorobenzo[b]thiophene-3-carboxylic acid, 6-fluorobenzo[b]thiophene-3-carboxylic acid, 4-hydroxybenzo[b]thiophene-3-carboxylic acid, 5-hydroxybenzo[b]thiophene-3-carboxylic acid, 6-hydroxybenzo[b]thiophene-3-carboxylic acid, 7-hydroxybenzo[b]thiophene-3-carboxylic acid, and benzo[b]thiophene-3-carboxylic acid. These carboxylic acids may have the substituents as defined above.

These carboxylic acids can be prepared in accordance with methods as described in Nippon Kagaku Zasshi Vo.I 88, No. 7, 758-763 (1967), Nippon Kagaku Zasshi Vol. 86, No. 10, 1067-1072 (1965), J. Chem. Soc (c) 1899-1905 (1967), J. Heterocycle. Chem. Vol. 10 679-681 (1973), J. Heterocyclic Chem. Vol 19 1131-1136 (1982) and J. Med. Chem. Vol. 29 1637-1643 (1986).

The reactive derivative of carboxylic acid of the formula (III) means the corresponding acid halide (e.g., chloride, brontide, iodide), acid anhydride (e.g., mixed acid anhydride with formic acid or acetic acid), active ester (e.g., succinimide ester), including acylaling agents used for the acylation of amino groups. For example, when an acid halide is employed, the compound (III) is reacted with a thionyl halide (e.g., thionyl chloride), phosphorous halide (e.g., phosphorous trichloride, phosphorous pentachloride), oxalyl halide (e.g., oxalyl chloride), in accordance with known methods as described in the literature (e.g., Shin Jikken-Kagaku-Koza, Vol. 14, 1787 (1978); Synthesis 852-854 (1986); Shin-Jikken-Kagaku-Koza Vol. 22, 115 (1992)).

The reaction of Process 1 can be conducted under conditions generally used for the acylation of amino groups. For example, in a case of condensation with the acid halide, the reaction is carried out in a solvent such as an ether solvent (e.g., diethyl ether, tetrahydrofuran, dioxane), benzene solvent (e.g., benzene, toluene, xylene), halogenated hydrocarbon solvent (e.g., dichloromethane, dichloroethane, chloroform) as well as ethyl acetate, dimethylformamide, dimethyl sulfoxide and acetonitrile, if necessary, in the presence of a base (e.g. an organic base such as triethylamine, pyridine, N,N-dimethylaminopyridine or N-methylmorpholine; an inorganic base such as sodium hydroxide, potassium hydroxide or potassium carbonate. The reaction temperature is that under cooling, at room temperature or under heating, preferably at a temperature ranging from - 20 °C to ice-cooling temperature or from room temperature to the refluxing temperature of the reaction system. The reaction time is several minutes to several tens of hours, preferably 0.5 hr to 24 hr and particularly 1 hr to 12 hr. When using the carboxylic acid in a free form without converting into the reactive derivative, the reaction is conducted in the presence of a condensing agent (e.g., dicyclohexylcarbodiimide (DCC), 1-ethyl-3-(3-methylaminopropyl)carbodiimide, N,N'-carbonyldiimidazole) usually used in the condensation reactions of amines with carboxylic acids.

The compound (I) can be also prepared in accordance with a method as follows.

Process 2

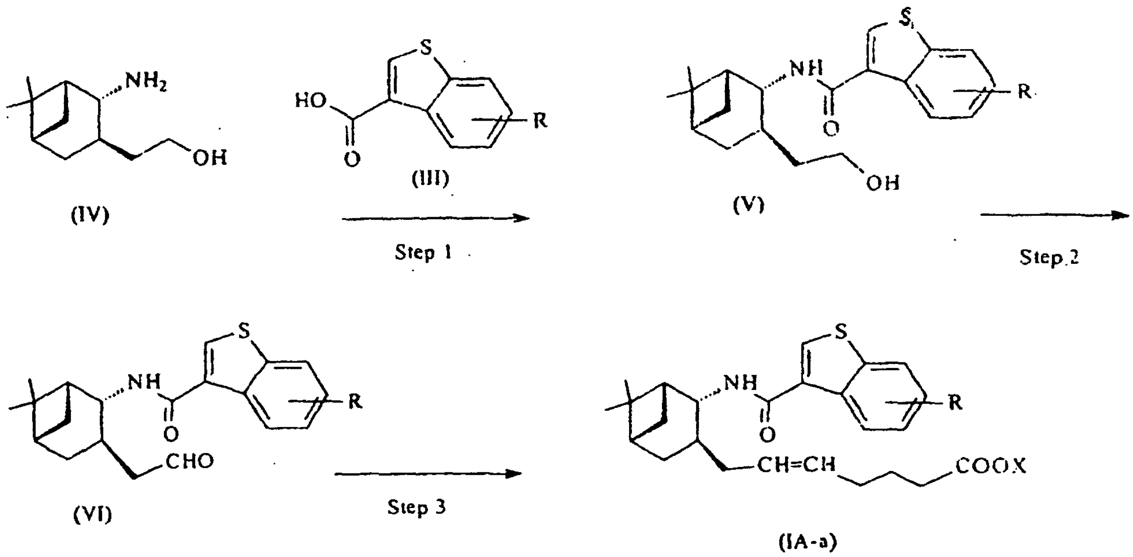


wherein R and X are as defined above and the double bond on the α-chain has Z configuration.

(Step 1)

In this step, the compound of the formula (V) can be prepared by reacting the amino compound of the formula (IV) with a carboxylic acid of the formula (III) or its reactive derivative, in accordance with Process 1. As to some of the amino compounds of the formula (IV), the process is disclosed in Chem. Pharm. Bull. Vol. 37, No. 6, 1524-1533 (1989).

(Step 2)

In this step, a compound of the formula (V) is oxidized to give an aldehyde compound of the formula (VI). This step may be carried out with chromated oxidizing agents such as Jones' reagent, Collins' reagent, pyridinium chlorochromate and pyridinium dichromate, in a solvent such as a chlorinated hydrocarbon (e.g., chloroform, dichloromethane), ether (e.g., ethyl ether, tetrahydrofuran), acetone, benzene, under cooling or at room temperature for several hours. This step may be also carried out with oxidizing agents in combination with appropriate activator agents (e.g., trifluoroacetic anhydride, oxalyl chloride) and dimethyl sulfoxide, if necessary, in the presence of a base (e.g. an organic base such as triethylamine, diethylamine).

(Step 3)

In this step, the $\alpha$ chain of an aldehyde compound of the formula (VI) is formed to give the compound of the formula (IA-a). The compound of the formula (IA-a) can be prepared by reacting the aldehyde compound of the formula (VI) with an ylide compound corresponding to the remaining part of the $\alpha$ chain in accordance with conditions of the Wittig reaction. Further, the ylide compound corresponding to the remaining part of the $\alpha$ chain can be synthesized by reacting triphenylphosphine with a corresponding halogenated alkanoic acid or ester derivative thereof in the presence of a base according to a well known method.

In a reaction of the other free acid or the reactive derivative with the amine (II) or (IV), depending on the property of each free acid or the reactive derivative, the reaction conditions are determined in accordance with a known method. The reaction product can be purified by a conventional method, such as extraction with a solvent, chromatography or recrystallization.

The objective compound (IA-a) in the present invention can be converted into a corresponding ester derivative, if desired. For example, the ester can be prepared by esterification of a carboxylic acid in accordance with a known method.

When using a $PGD_2$ antagonist in the manufacture of a medicament according to the present invention for treatment, the $PGD_2$ antagonist can be formulated into ordinary formulations for oral and parenteral administration. A pharmaceutical composition containing a $PGD_2$ antagonist for use in the present invention can be in the form for oral and parenteral administration. Specifically, the oral formulation includes tablets, capsules, granules, powders, syrup. The parenteral formulation includes injectable solutions or suspensions for intravenous, intramuscular or subcutaneous injection, inhalants, eye drops, nasal drops, suppositories or percutaneous formulations such as ointment, patches and poultices. Preferred is an oral or percutaneous formulation.

In preparing the formulations, carriers, excipients, solvents and bases known to persons ordinarily skilled in the art may be used. Tablets are prepared by compressing or formulating an active ingredient together with auxiliary components. Examples of the auxiliary components include pharmaceutically acceptable excipients such as binders (e.g., cornstarch), fillers (e.g., lactose, microcrystalline cellulose), disintegrants (e.g., starch sodium glycolate) and lubricants (e.g.. magnesium stearate). Tablets may be coated appropriately. In the case of liquid formulations such as syrups, solutions or suspensions, they may contain suspending agents (e.g., methyl cellulose), emulsifiers (e.g., lecithin), preservatives. Injectable formulations may be in the form of solution or suspension or oily or aqueous emulsion, which may contain a suspension-stabilizing agent or dispensing agent. Percutaneous formulations such as ointments, patches, poultices and the like may be prepared by using water base (e.g., water, lower alcohol, polyol) or oil base (higher fatty acid ester (isopropyl myristate), lipophilic alochol).

An appropriate dosage of $PGD_2$ receptor antagonist, depending on the administration route, age, body weight, sex or condition of the patient and the kind of optionally combined drug(s), should be determined by a physician. In the case of oral administration, the daily dosage can generally be between about 0.01 - 100 mg, preferably about 0.01 - 10 mg, more preferably about 0.01 - 1 mg, per kg body weight. In the case of parenteral administration, the daily dosage can generally be between about 0.001 - 100 mg, preferably about 0.001 - 1 mg, more preferably about 0.001 - 0. 1 mg, per kg body weight. The daily dosage can be administered in 1 - 4 divisions.

The following Examples are provided to further illustrate the present invention and are not to be construed as limiting its scope.

The abbreviations used throughout the examples are shown as follows.

Me methyl
Ac acetyl
Ph phenyl

Reference 1

Preparation of 5-Benzenesulfonyloxybenzo[b]thiophene-3-carbonyl chloride (3)

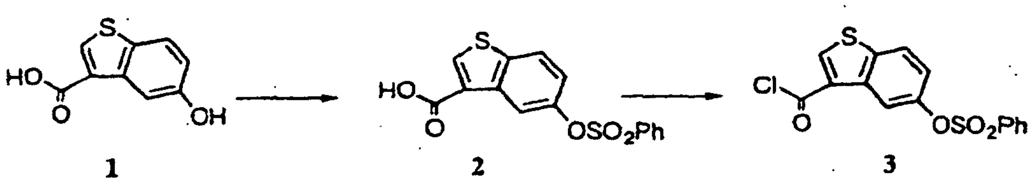

1 2 3

To a solution of 8.63 g (44.4 mmol) of 5-hydroxybenzo[b]thiophene-3-carboxylic acid (1) (J.Chem.Soc (C), 1899-1905 (1967), M.Martin-Smith et al.) in 160 ml of 80 % aqueous tetrahydrofuran and 44 ml of 1N sodium hydroxide were added dropwised 87 ml of 0.56N sodium hydroxide and 6.2 ml ( 48.4 mmol) of benzenesulfonylchloride simultaneously with maintaining pH 11-12 and stirring under ice-cooling. After the reaction, the mixture was diluted with water, alkalized and washed with toluene. The aqueous layer was weakly acidified with conc. hydrochloric acid under stirring. The precipitated crystals were filtered, washed with water and dried to give 14.33 g of 5-benzenesulfonyloxybenzo[b]thiophene-3-carboxylie acid (2).
mp 202-203 °C.
NMR δ ($CDCl_3$),300MHz
7.16 (1H, dd, J=2.7 and 9.0Hz), 7.55-7.61 (2H, m), 7.73 (1H, m), 7.81 (1 H,d, J=9.0Hz), 7.90-7.94(2H,m),8.16(1H,d, J=2.7Hz),8.60(1H,s).
IR(Nujol): 3102, 2925, 2854, 2744, 2640, 2577, 1672, 1599, 1558, 1500, 1460, 1451 $cm^{-1}$

| Elemental analysis (for $C_{15}H_{10}O_5S_2$) | | | |
|---|---|---|---|
| Calcd. (%) | C,53.88; | H,3.01; | S,19.18 |
| Found (%) | C,53.83; | H,3.03; | S,19.04 |

A mixture of 5.582 g (16.7 mmol) of the above obtained 5-benzenesulfonyloxybenzo[b]thiophene-3-carboxylic acid (2), a drop of dimethylformamide, 3.57 ml (50 mmol) of thionyl chloride and 22 ml of toluene was refluxed for 1.5 hours and then concentrated under reduced pressure to give 5.89 g of the objective compound (3).

Reference 2

Preparation of 5-Acetoxybenzo[b]thiophene-3-carbonyl chloride (5)

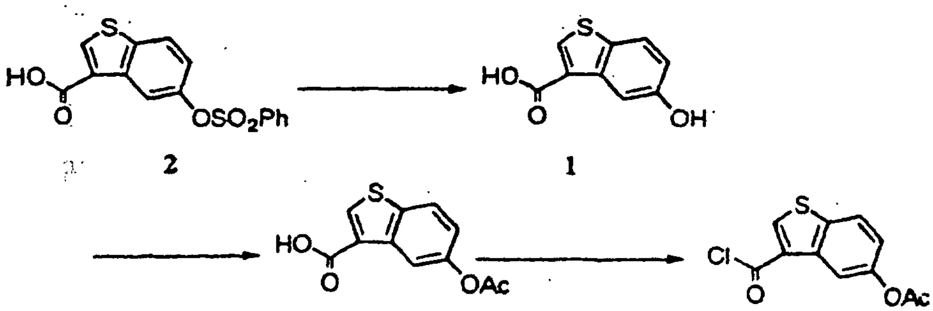

2 1 4 5

A solution of 100 mg (0.3 mmol) of the above obtained 5-benzenesulfonyloxybenzo[b]thiophene-3-carboxylic acid (2) in 1.2 ml of 1N sodium hydroxide was stirred at 40 °C for 8 hours. Hydrochloric acid (1N 1.2 ml) was added thereto and the precipitated crystals were filtered, washed with water and dried to give 58 mg of 5-hydroxybenzo[b]thiophene-3-carboxylic acid (1). Yield 96.6 %.
mp 262-263 °C

A solution of 1,140 mg of the above obtained 5-hydroxybenzo[b]thiophene-3-carboxylic acid (1) in 2 ml of acetic anhydride and 4 ml of pyridine was allowed to stand for 3 hours. After addition of water, the mixture was stirred for 1.5 hours under ice-cooling and the precipitated crystals were filtered, washed with water and dried to give 1,349mg of 5-acetoxybenzo[b]thiophene-3-carboxylic acid (4). Yield 97.3 %.
mp 239-240 °C.

A mixture of 1,349 mg of the above obtained 5-acetoxybenzo[b]thiophene-3-carboxylic acid (4), a drop of dimethylformamide, 1.22 ml of (17.13 mmol) of thionyl chloride and 25 ml of toluene was refluxed for 1.5 hours and then concentrated under reduced pressure to give 1,454 mg of the objective compound (5).

Reference 3

Preparation of (1R, 2S, 3S, 5S)-2-(2-Amino-6,6-dimethylbicyclo[3.1.1]hept-3-yl)ethanol (IVA-b-1) and (1R, 2R, 3S, 5S)-2-(2-Amino-6,6-dimethylbicyclo[3.1.1]hept-3-yl)ethanol (IVA-c-1)

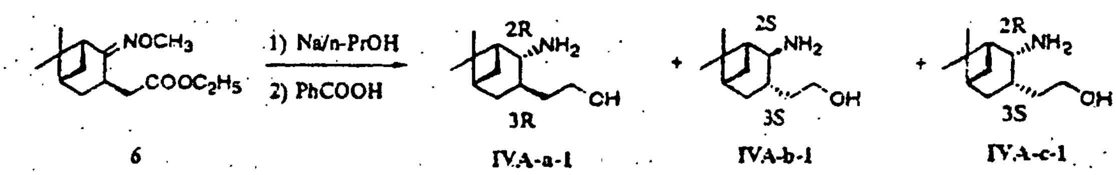


6 IVA-a-1 IVA-b-1 IVA-c-1

The compound (6) (Chem. Pharm. Bull. Vol.37, No.6 1524-1533 (1989)) was reduced with sodium according to the method described in the above literature and the compound (IVA-a-1) was removed by filtration as the benzoic acid salt. The mother liquor (79 g) was suspended in 150ml of ethyl acetate, adding 260 ml of 1N-hydrochloric acid and the mixture was stirred. The separated aqueous layer was basified with 65 ml of 4N-sodium hydroxide and extracted with ethyl acetate. The organic layer was washed with water, dried over anhydrous magnesium sulfate and concentrated under reduced pressure. Of the obtained oily residue (30 g), 6.7 g was dissolved in 40 ml of 90 % methanol, adsorbed to 500 ml of an ion-exchange resin, Amberlite CG-50 ($NH_4^+$)type I and eluted with 2.2 L of water and 1N 2.2 L of

aqueous ammonia by a gradient method. One fraction: 300ml. Each fraction was checked by thin-layer chromatography (the developing solvent ; chloroform: methanol: conc. aqueous ammonia= 90: 10: 1). The fractions 3-8 were collected and concentrated under reduced pressure. The residue was crystallized from hexane; recrystallization afforded 538 mg of needles.
mp 117-118 °C.
NMR δ ($CDCl_3$), 300MHz
1.01 and 1.21 (each 3H, each s), 1.34 (1H, d, J=9.9Hz), 1.52-1.66 (2H,m), 1.90-2.07 (4H,m), 2.18 (1H, m), 2.48 (1H, m), 3.12 (3H, bs), 3.49 (1H, d d,J=3.9 and 9.6Hz), 3.61 (1H, dt, J=2.4 and 10.5Hz). 3.84 (1H, ddd, J=3. 3, 4.8 and 10.5Hz).
IR(Nujol): 3391, 3293, 3108, 2989, 2923, 2869, 2784, 2722, 2521, 1601, 1489, 1466 $cm^{-1}$
$[\alpha]_D^{23}$·2.5° (c=1.02,$CH_3OH$)

| Elemental analysis for ($C_{11}H_{21}NO$) | | | |
|---|---|---|---|
| Calcd.: (%) | C,72.08; | H,11.55; | N,7.64 |
| Found: (%) | C,72.04; | H,11.58; | N,7.58 |

By means of X-ray crystal analysis, the obtained compound was identified as (1R, 2R, 3S, 5S)-2-(2-amino-6,6-dimethylbicyclo[3.1.1]hept-3-yl)ethanol (IVA-c-1). The mother liquor (2.9 g) after the recrystallization from hexane was dissolved in 15 ml of ethyl acetate, to which was added a solution of 30 ml of ethyl acetate containing 1.93 g of benzoic acid . The precipitated crystals were filtered to give 2.93 g of the benzoic acid salt of the compound (IVA-a-1). mp 182-183 °C.

The fractions 10-17 were collected and concentrated under reduced pressure. To a solution of 2.66 g of the residue in 15 ml of ethyl acetate was added 11 ml of ethyl acetate containing 1.77 g of benzoic acid. The precipitated crystals were filtered to give 4.08 g of needles.
mp 160-161 °C.
NMR δ ($CDCl_3$),300MHz
0.61 and 1.06 (each 3H, each s), 1.36 (1H, m), 1.53-1.65 (2H, m), 1.75-1.88(2H,m), 1.95-2.04 (4H, m), 3.18 (1H, d, J=6.3Hz), 3.58 (1H, dt, J=3.0 and 10.8Hz), 3.81 (1H, m), 5.65 (4H, bs), 7.33-7.42 (3H, m), 7.98-8.01 (2H, m).
IR(Nujol): 3320, 2922, 2854, 2140, 1628, 1589, 1739, 14591389 $cm^{-1}$
$[\alpha]_D^{23}$·31.8° (c=1,01,$CH_3OH$)

| Elemental analysis (for $C_{18}H_{27}NO_3$) | | | |
|---|---|---|---|
| Calcd.: (%) | C,70.79; | H,8.91; | N,4.59 |
| Found: (%) | C,70.63; | H,8.86; | N,4.58 |

By means of X-ray crystal analysis, the structural formula was identified as that of (1R, 2S, 3S, 5S)-2-(2-amino-6,6-dimethylbicyclo[3.1.1]hept-3-yl)ethanol (IVA-b-1).

Example 1

Preparation of Sodium (5Z)-7-{(1R,2R,3S,5S)-2-(5-hydroxybenzo[b]thiophen-3-yl-carbonylamino)-6,6-dimethylbicyclo[3.1.1]hept-3-yl}-5-heptenoate (IA-a-2)

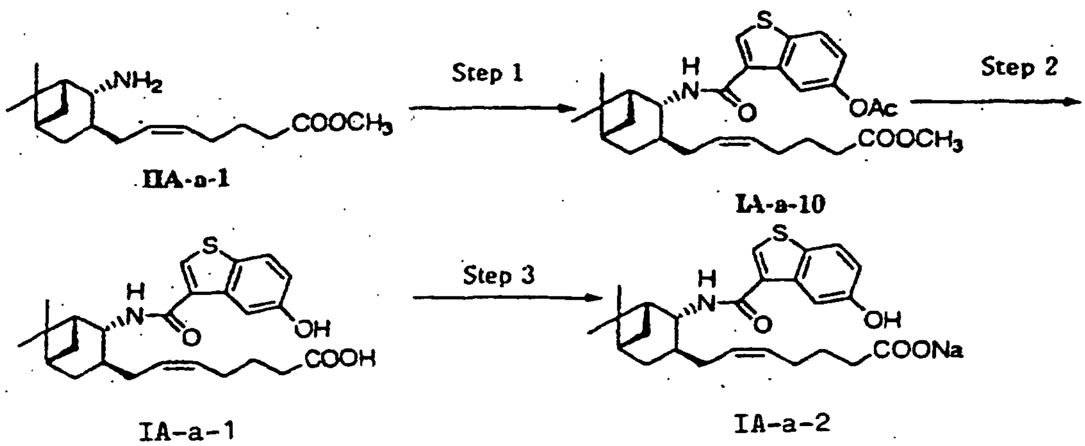


IIA-a-1 IA-a-10 IA-a-1 IA-a-2

(Step 1)

To a solution of 1,450 mg (5.2 mmol) of the compound (IIA-a-1) (Japanese Patent Publication (Kokoku) No. 23170/1994) in 25 ml of tetrahydrofuran were added 2.6 ml (18.7 mmol) of triethylamine and 1,454 mg (1.1 mmol) of 5-acetoxybenzo[b]thiophene-3-carbonyl chloride (5) obtained in Reference 2. After stirring for 1.5 hours, the mixture was diluted with water and extracted with toluene. The organic layer was washed with dilute hydrochloric acid and water, dried over anhydrous magnesium sulfate and concentrated under reduced pressure. The residue was chromatographed on silica gel (toluene:ethyl acetate=9:1) to give 2,481 mg of the compound (IA-a-10). Yield 96.1 %.
$[\alpha]_D^{23}$=+48.0° (c=1.01%,$CH_2OH$)

| Elementary Analysis (for $C_{28}H_{35}NO_5S \cdot 0.1H_2O$) | | | | |
|---|---|---|---|---|
| Calcd.(%) | C,67.34; | H,7.10; | N,2.80; | S,6.42 |
| Found(%) | C,67.23; | H,7.12; | N,2.86; | S,6.59 |

(Step 2)

To a solution of 2,357 mg (4.73 mmol) of the above obtained compound (IA-a-10) in 25 ml of methanol was added 4.1 ml (16.4 mmol) of 4N sodium hydroxide. After stirring for 6 hours, the mixture was neutralized with 17 ml of 1N hydrochloric acid, diluted with water and extracted with ethyl acetate. The organic layer was washed with water, dried over magnesium sulfate and concentrated under reduced pressure. The residue was recrystallized from ethyl acetate/n-hexane to give 1,859 mg of the compound (IA-a-1) as prisms. Yield 86.5 %.
mp 142-143 °C.
$[\alpha]_D^{23}$=+47.6° (c=1.01%,$CH_3OH$)

| Elementary Analysis (for $C_{25}H_{31}NO_4S$) | | | | |
|---|---|---|---|---|
| Calcd.(%) | C,68.00; | H,7.08; | N,3.17; | S,7.26 |
| Found(%) | C,67.93; | H,7.08; | N,3.19; | S,7.24 |

(Step 3)

To a solution of 203 mg (0.46 mmol) of the above obtained compound (IA-a-1) in 3 ml of methanol was added 0.42 ml (0.42 mmol) of 1N sodium hydroxide and the mixture was concentrated under reduced pressure. The residue

was dissolved in a small quantity of ethyl acetate and diluted with n-liexane. Theinsolublematerial was dissolved in methanol and concentrated under reduced pressure to give 210 mg of the objective compound (IA-a-2) Yield 98.5 %. $[\alpha]_D^{25}$=+38.9° (c=1.00%,$CH_3OH$)

| Elemental analysis (for $C_{35}H_{30}NO_4SNa \cdot 0.5H_2O$) | | | | | |
|---|---|---|---|---|---|
| Calcd.(%) | C,63.54; | H,6.61; | N,2.96; | S,6.78; | N a,486 |
| Found(%) | C,63.40; | H,6.69; | N,3.13; | S,6.73; | N a,4.68 |

Compounds and physical constants obtained in the same manner as the above Examples are shown in the following table 1 -table 3.

Table 1

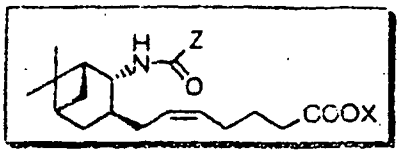

| Compd.No. | X | Z | Compd.No. | X | Z |
|---|---|---|---|---|---|
| IA-a-1 | H | 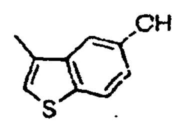 | IA-a-5 | H | 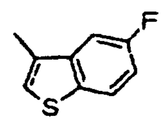 |
| IA-a-2 | Na | 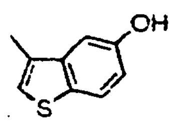 | IA-a-6 | H | 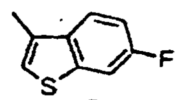 |
| IA-a-3 | H | 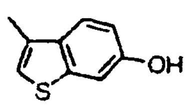 | IA-a-7 | H | 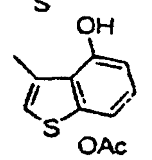 |
| IA-a-4 | H | 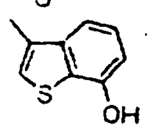 | IA-a-8 | H | 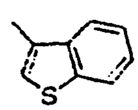 |

Table 2

| Compd. No. | Physical property |
|---|---|
| IA-a-1 | 1.02 (1H,d,J=10.2Hz),1.12 and 1.24 (each 3H,each s), 1.56-2.55 (14H,m), 4.29 (1H,m), 5.32-5.51 (2H,m),<br>6.20 (1H,d,J=9.3Hz), 7.01 (1H,dd,J=2.4 and 9.0Hz), 7.66 (1H,d,J=9.0Hz), 7.69 (1H,s), 8.03 (1H,d, J=2.4Hz).<br>IR ($CHCl_3$): 3600, 3440, 3226, 1707, 1638, 1602, 1516 $cm^{-1}$.<br>[α]D +47.6° ($CH_3OH$, c=1.00,23°). mp 142-143°C |
| IA-a-2 | ($CD_3OD$) 0.97 (1H,d,J=9.9Hz), 1.16 and 1.25 (each 3H,each s), 1.55-2.43 (14H,m), 4.18 (1H,m), 5.41-5.53 (2H,m), 6.93 (1H,dd,J=0.6 and 8.7Hz), 7.G8 (1H,dd,0.6 and 8.7Hz). 7.71 (1H,m), 8.01 (1H,s).<br>IR (KBr): 3436, 2621, 1637, 1600, 1557, 1520, 1434$cm^{-1}$.<br>[α]D +38.9° ($CH_3OH$, c=1.00,25°C). |
| IA-a-3 | 0.97 (1H.d,J=10.2Hz), 1.10 and 1.23 (each 3H,each s), 1.54-2.52 (14H,m), 4.32 (1H,m), 5.35-5.54 (2H,m), 6.26 (1H,d,J=8.7Hz), 6.98 (1H,dd,J=2.4 and 9.0Hz), 7.26 (1H,m), 7.58 (1H,s), 8.07 (1H,d, J=9.0Hz).<br>IR ($CHCl_3$): 3592, 3439, 3223, 3102, 1708, 1639, 1604, 1518$cm^{-1}$.<br>[α]D +51.5° ($CH_3OH$, c=1.01,25°C). |
| IA-a-4 | 0.96 (1H,d,J=10.2Hz), 1.11 and 1.24 (each 3H,each s). 1.54-2.53 (14H,m), 4.34 (1H,m), 5.35-5.53 (2H,m), 6.31 (1H,d,J=9.0Hz), 6.79 (1H,d,J=7.5Hz), 7.25 (1H,dd,J=7.5 and 8.4Hz), 7.74 (1H,d, J=8.4Hz), 7.86 (1H,s).<br>IR ($CHCl_3$): 3586, 3437, 3104, 1708, 1638, 1568, 1522, 1501, 1471 $cm^{-1}$. [α]D +57.1° ($CH_3OH$, c=1.01,25°C). |

Table 3

| | |
|---|---|
| IA-a-8 | NMR δ ($CDCl_3$), 300MHz<br>1.00(1H,d,J=10.5Hz),1.12 and 1.23(each 3H,each s),1.50-1.66(3H,m), 1.84-2.03(4H,m),2.17-2.40(7H,m), 4.33(1H,m), 5.42- 5.45(2H,m),6.16(1H,d,J=9.0Hz),7.01(1H,dd,J=2.4 and 8.7Hz),7.66(1H,d,J=8.7Hz), 7.69(1H,s), 8.04(1H,d,J=2.4Hz).<br>IR($CHCl_3$):3441,3237,3035,3009,2992,2924,2870,1708,163 7,1601,1516,1436 $cm^{-1}$<br>$[\alpha]_D^{24}$+14.4° (c=1.01%,$CH_3OH$) |

The compounds prepared in the Examples above were tested as shown in the Experimental examples below.

Experiment 1 Binding to $PGD_3$ Receptor

Materials and Methods

(1) Preparation of Human Platelet Membrane Fraction

A blood sample was obtained using a plastic syringe containing 3.8 % sodium citrate from the vein of healthy volunteers (adult male and female), put into a plastic test tube and mixed gently by rotation. The sample was then centrifuged at 1800 rpm, 10 min at room temperature and the supernatant containing PRP (platelet-rich plasma) was collected. The PRP was recentrifuged at 2300 rpm, 22 min at room temperature to obtain platelets. The platelets were homogenized using a homogenizer (Ultra-Turrax) followed by centrifugation 3 times at 20,000 rpm, 10 min at 4°C to obtain a platelet membrane fraction. After protein determination, the membrane fraction was adjusted to 2 mg/ml and preserved in a refrigerator at -80°C until use.

(2) Binding to $PGD_2$ Receptor

To a binding-reaction solution (50 mM Tris/HCl, pH 7.4, 5 mM $MgCl_2$) (0.2 ml) were added the human platelet membrane fraction (0.1 mg) and 5 nM $[^3H]PGD_2$ (115Ci/mmol). After reacting at 4°C for 90 min, the mixture was filtered through a glass fiber filter paper and washed several times with cooled saline, to measure the radioactivity retained

on the filter paper. The specific binding was calculated by subtracting the non-specific binding (the binding in the presence of 10 μM $PGD_2$) from the total binding. The inhibitory activity of each compound was expressed as the concentration required for 50 % inhibition ($IC_{50}$), which was determined by depicting a substitution curve by plotting the binding ratio (%) in the presence of each compound, where the binding ratio in the absence of a test compound is 100 %. The results are shown in Table 4.

Table 4

| Compound No. | $IC_{50}$ (nM) |
|---|---|
| | |
| IA-a-1 | 0.4 |
| IA-a-3 | 1.3 |
| | |
| IA-a-5 | 0.27 |
| IA-a-8 | 32 |

Experiment 2 Investigation on itch-related scratching behavior in mouse pruritus model; Effect on compound 48/80-induced scratching behavior.

Compound 48/80 (10 μg/site, Sigma), a non-immunological mast cell activator, was dissolved in physiological saline and subcutaneously injected into the rostral part of the back of female C57BL mice (8-12 weeks of age, Charles River Japan Inc.). After the injection, scratching behavior was observed for 30 minutes. Mice usually scrateched several times successively in one behavior and a series of scratching was counted as one incidence.

Compound treatment: Compound (IA-a-1), suspended in 0.5% methylcellulose, was orally administered 1 hour before the injection of compound 48/80 at a dose of 300 mg/kg. As a control, mice were treated with 0.5% methylcellulose. Results were shown in Table 5. $P < 0.05$ versus control (Wilcoxon test).

Table 5

| | Mean ± S.E. (Number of scratching) |
|---|---|
| Control (n = 10) | 122.2 ± 12.7 |
| Compd. (IA-a-5) 300 mg/kg (n = 10) | 81.8 ± 17.9 * |
| (Wilcoxon test, P < 0.05) | |

Experiment 3. Investigation on itch-related scratching behavior in mouse pruritus model: Effect on antigen-induced scratching behavior.

50 μl of diluted anti-benzylpenicilloyl (BPO) IgE monoclonal amibody was intradermally injected to the rostral part of the back skin of female C57BL (Table 6 , 8-12 weeks of age, Charles River Japan Inc.) or male DS-Nh (Table 18, 7-8 weeks of age, Aburahi Laboratories, Shionogi & Co., Ltd.) mice. Twenty-four hours later, scratching behavior was induced by intravenously injection of physiological saline containing 1 mg of BPO-guinea pig serum albumin. Scratching behavior was observed for 15 minutes (Table 6 ') or 10 minutes (Table 7 ) as described above.

In Table 6 the compound was treated in the same manner described in 1) above, In Table 7, the compound were dissolved in physiological saline and intraperitoneally injected 30 minutes before antigen challenge. *: $P < 0.05$, **: $P < 0.01$ versus control (Dunnett's test).

Table 6

| | Mean ± S.E. (Number of scratching) |
|---|---|
| Control (n = 7) | 51.6 ± 5.3 |
| Compd. (IA-a-5) 100 mg/kg (n = 7) | 48.4 ± 12.4 |
| Compd. (IA-a-5) 300 mg/kg (n = 7) | 19.6 ± 6.5 * |
| (Dunnett's test, P < 0.05) | |

Table 7

| | Mean ± S.E. (Number of scratching) |
|---|---|
| Control (n = 5) | 47.8 ± 8.0 |
| Compd. (IA-a-17) Na salt 100 mg/kg (n = 5) | 11.6 ± 3.0 ** |
| Compd. (IA-a-11) Na salt 100 mg/kg (n = 5) | 4.4 ± 1.9 ** |
| Compd. (IA-a-7) Na salt 100 mg/kg (n = 5) | 29.2 ± 11.6 |
| Control (n = 6) | 52.3 ± 7.2 |
| Compd. (IA-a-11) Na salt 10 mg/kg (n = 6) | 43.7 ± 6.2 |
| Compd. (IA-a-11) Na salt 30 mg/kg (n = 6) | 40.0 ± 5.4 |
| Compd. (IA-a-11) Na salt 100 mg/kg (n = 6) | 18.2 ± 3.3 ** |
| (Dunnett's test, P < 0.01) | |

As shown in Experiment 2, scratching caused by skin itching derived from the activation of mast cell was reduced in the test group of a compound (IA-a-1) as compared with the control group. As shown in Experiment 3, scratching of the test group, caused by skin itching derived from the antigen-stimulation, was reduced as compared with the control group.

As shown above, a compound of the present invention is useful as a pharmaceutical composition for preventing or treating diseases accompanied by itching derived from allergic reaction or a reaction similar thereto, for example, atopic dermatitis, urticaria, atopic conjunctivitis, allergic rhinitis and contact dermatitis. Moreover, the present compound is applicable to a pharmaceutical composition for preventing or treating secondary diseases such as cataracts, retinal separation, inflammation, infection, dysgryphia, caused by an action accompanied by itching, for example, scratching, knocking.

| Formulation example 1 | tablet |
|---|---|
| compound (IA-a-1) | 40.0 mg |
| hydroxypropyl cellulose | 3.6 mg |
| magnesium stearate | 0.4 mg |
| corn starch | 18.0 mg |
| saccharum lactis | 58.0 mg |
| | Total 120.0 mg |

| Formulation example 2 | unguentum |
|---|---|
| compound (IA-a-1) | 0.1 g |
| liquid paraffin | 1.5 g |
| white petrolatum | 18.4 g |
| | Total 20.0 g |

Industrial Applicability

As shown clearly in the above experiments, a compound of the present invention has an activity for the prevention or treatment of itching. Therefore, the compound of the present invention is useful as a pharmaceutical composition for the prevention or treatment of itching and is applicable to a pharmaceutical composition for preventing or treating diseases accompanied by itching, for example, atopic dermatitis, urticaria, allergic conjunctivitis, allergic rhinitis, contact dermatitis. Moreover, the present compound is applicable to a pharmaceutical composition for the prevention or treatment of a secondary disease such as cataracts, retinal separation, inflammation, infection, dysgryphia, caused by an action accompanied by itching, for example, scratching, knocking.

## Claims

**1.** Use of a compound of the formula (IA-a):

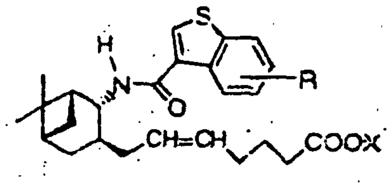

(IA-a)

wherein R is hydrogen, fluoro or hydroxy and X is hydrogen and the double bond on the $\alpha$ chain has Z configuration, a pharmaceutical acceptable salt thereof or a hydrate thereof, in the manufacture of a medicament for the treatment or prevention of itching.

**2.** Use of a compound of the formula (IA-a) according to claim 1, wherein R is hydroxy and X is hydrogen.

**3.** Use of a compound according to claim 2, wherein the compound is of the formula (IA-a-1):

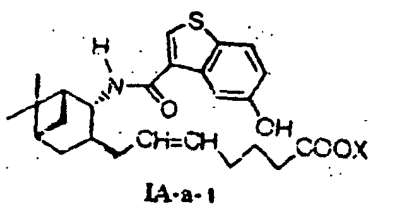

IA-a-1

wherein X is hydrogen, a pharmaceutical acceptable salt thereof or a hydrate thereof.

**4.** Use of a compound according to any one of claims 1 to 3, wherein the itching is caused by an antigen.

**5.** Use of a compound according to any one of claims 1 to 3, wherein the itching is derived from atopic dermatitis.

**6.** Use of a compound according to any one of claims 1 to 3, wherein the itching is derived from urticaria.

**7.** Use of a compound according to any one of claims 1 to 3, wherein the itching is derived from allergic conjunctivitis.

**8.** Use of a compound according to any one of claims 1 to 3, wherein the itching is derived from allergic rhinitis.

**9.** Use of a compound according to any one of claims 1 to 3, wherein the itching is derived from contact dermatitis.

## Patentansprüche

**1.** Verwendung einer Verbindung der Formel (IA-a):

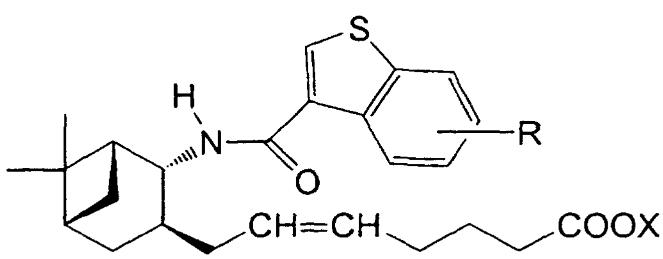

(IA-a)

worin R Wasserstoff, Fluor oder Hydroxy ist und X Wasserstoff ist und die Doppelbindung an der α-Kette Z-Konfiguration hat, eines pharmazeutisch annehmbaren Salzes davon oder eines Hydrates davon bei der Herstellung eines Medikaments für die Behandlung oder Prävention von Juckreiz.

2. Verwendung einer Verbindung der Formel (IA-a) nach Anspruch 1, wobei R Hydroxy und X Wasserstoff ist.

3. Verwendung einer Verbindung nach Anspruch 2, wobei die Verbindung die Formel (IA-a-1) hat:

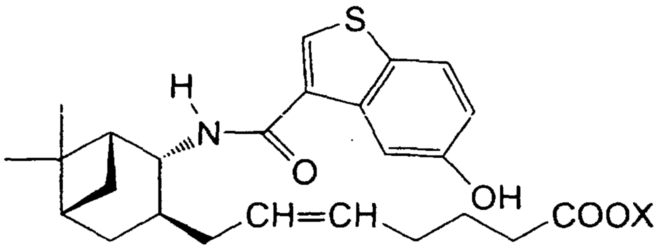

(IA-a-1)

worin X Wasserstoff ist, eines pharmazeutisch annehmbaren Salzes davon oder eines Hydrates davon.

4. Verwendung einer Verbindung nach einem der Ansprüche 1 bis 3, wobei der Juckreiz durch ein Antigen verursacht wird.

5. Verwendung einer Verbindung nach einem der Ansprüche 1 bis 3, wobei der Juckreiz von atopischer Dermatitis herrührt.

6. Verwendung einer Verbindung nach einem der Ansprüche 1 bis 3, wobei der Juckreiz von Urticaria herrührt.

7. Verwendung einer Verbindung nach einem der Ansprüche 1 bis 3, wobei der Juckreiz von allergischer Konjunktivitis herrührt.

8. Verwendung einer Verbindung nach einem der Ansprüche 1 bis 3, wobei der Juckreiz von allergischer Rhinitis herrührt.

9. Verwendung einer Verbindung nach einem der Ansprüche 1 bis 3, wobei der Juckreiz von Kontaktdermatitis herrührt.

## Revendications

1. Utilisation d'un composé de formule (IA-a) :

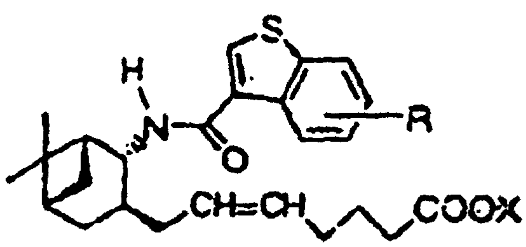

(IA-a)

dans laquelle R représente un atome d'hydrogène, un groupe fluoro ou hydroxy et X représente un atome d'hydrogène, et la double liaison sur la chaîne α possède la configuration Z, d'un de ses sels pharmaceutiquement acceptables ou d'un de ses hydrates dans la production d'un médicament destiné au traitement ou à la prévention du prurit.

2. Utilisation d'un composé de formule (IA-a) suivant la revendication 1, dans laquelle R représente un groupe hydroxy et X représente un atome d'hydrogène.

3. Utilisation d'un composé suivant la revendication 2, dans laquelle le composé répond à la formule (IA-a-1) :

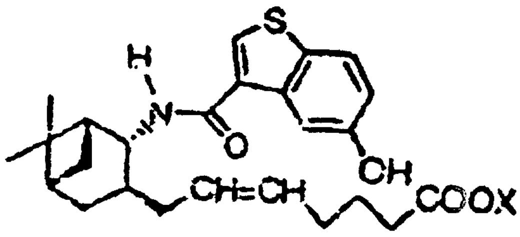

IA-a-1

dans laquelle X représente un atome d'hydrogène, d'un de ses sels pharmaceutiquement acceptables ou d'un de ses hydrates.

4. Utilisation d'un composé suivant l'une quelconque des revendications 1 à 3, dans laquelle le prurit est provoqué par un antigène.

5. Utilisation d'un composé suivant l'une quelconque des revendications 1 à 3, dans laquelle le prurit est dû à une dermatite atopique.

6. Utilisation d'un composé suivant l'une quelconque des revendications 1 à 3, dans laquelle le prurit est dû à une urticaire.

7. Utilisation d'un composé suivant l'une quelconque des revendications 1 à 3, dans laquelle le prurit est dû à une conjonctivite allergique.

8. Utilisation d'un composé suivant l'une quelconque des revendications 1 à 3, dans laquelle le prurit est dû à une rhinite allergique.

9. Utilisation d'un composé suivant l'une quelconque des revendications 1 à 3, dans laquelle le prurit est dû à une dermatite de contact.